Europäisches Patentamt

European Patent Office

Office européen des brevets

(19)

(11) Veröffentlichungsnummer : **0 011 335**
**B1**

(12) # EUROPÄISCHE PATENTSCHRIFT

(45) Veröffentlichungstag der Patentschrift :
28.04.82

(21) Anmeldenummer : 79200649.6

(22) Anmeldetag : 06.11.79

(51) Int. Cl.³ : **H 01 F 7/20**, G 01 N 24/04,
A 61 B 5/05

(54) Magnetspulenanordnung zur Erzeugung eines homogenen Magnetfeldes.

(30) Priorität : 14.11.78 DE 2849355

(43) Veröffentlichungstag der Anmeldung :
28.05.80 (Patentblatt 80/11)

(45) Bekanntmachung des Hinweises auf die Patenterteilung : 28.04.82 Patentblatt 82/17

(84) Benannte Vertragsstaaten :
BE DE FR GB IT NL

(56) Entgegenhaltungen :
DE - A - 1 566 148
DE - B - 1 049 007
TECHNISCHE MITTEILUNGEN KRUPP, FORSCHUNGSBERICHTE, Vol. 29, Nr. 3/4, 1971, Dezember,
Essen, DE
H. ESCHENAUER et al. : « Über die Projekt-Definitionsphase des mechanischen Systems einer Magnetfeldsimulationsanlage », Seiten 119-132
TECHNISCHE MITTEILUNGEN KRUPP, WERKSBERICHTE, Vol. 32, Nr. 3, 1974
Essen, DE
H. ESCHENAUER et al. : « Über die Entwicklungs- und Fertigungsphase des mechanischen Systems der Magnetfeldsimulationsanlage MFSA II in Ottobrunn », Seiten 105-114
REVIEW OF SCIENTIFIC INSTRUMENTS,
American Institute of Physics, Vol. 46, Nr. 9, September 1975,
New York, US.

(73) Patentinhaber : Philips Patentverwaltung GmbH
Steindamm 94
D-2000 Hamburg 1 (DE)
DE
N.V. Philips' Gloeilampenfabrieken
Pieter Zeemanstraat 6
NL-5621 CT Eindhoven (NL)
BE FR GB IT NL

(72) Erfinder : Heinzerling, Jürgen, Dr.
Pezolddamm 42
D-2000 Hamburg 71 (DE)
Erfinder : Rieckeheer, Rainer, Dr.
Drieblad-Hof Nr.5
NL-5672 XH Nuenen (NL)

(74) Vertreter : Hartmann, Heinrich, Dipl.-Ing. et al
Philips Patentverwaltung GmbH Steindamm 94
D-2000 Hamburg 1 (DE)

(56) Entgegenhaltungen :
C.B. JOHNSON : « Tri-Coil electromagnet », Seiten 1289-1290
J. APPL Phys. Vol. 22, Sept. 1951, S. 1091-1107
REVIEW OF SCIENTIFIC INSTRUMENTS Vol. 33 (1962) S. 933-938

Imprimerie Jouve, 18, rue St-Denis, 75001 Paris, France

## Magnetspulenanordnung zur Erzeugung eines homogenen Magnetfeldes

Die Erfindung betrifft eine Magnetspulenanordnung zur Erzeugung eines wenigstens in ihrem Zentrum homogenen Magnetfeldes, vorzugsweise für die Magnetresonanz-Spektroskopie, bestehend aus vier ebenen, paarweise gleichen Ringspulen, von denen zu verschiedenen Paaren gehörende Ringspulen, die mit ihren Spulenebenen senkrecht zu einer durch die Spulenmittelpunkte hindurchtretenden Untersuchungsachse liegen und symmetrisch zu einem Zentralpunkt auf der Untersuchungsachse angeordnet sind, unterschiedliche mittlere Wicklungsdurchmesser sowie rechteckförmige Wicklungsquerschnitte besitzen, deren Seiten senkrecht bzw. parallel zur Untersuchungsachse verlaufen, wobei das Spulenpaar mit dem grösseren mittleren Wicklungsdurchmesser zwischen dem mit dem kleineren mittleren Wicklungsdurchmesser liegt und alle Ringspulen in gleicher Richtung vom Strom durchflossen werden.

Eine derartige Magnetspulenanordnung ist z.B. aus dem Aufsatz « Axially Symmetrie Systems for Generating and Measuring Magnetic Fields » von M. W. Garrett, erschienen in Appl. Phys., Sept. 1951, vol. 22 s. 1091-1107, bekannt. Mit ihr läßt sich unter der Voraussetzung verschwindend kleiner Wicklungsquerschnitte ein Magnetfeld B erzeugen, welches rotationssymmetrisch und in Richtung einer Untersuchungsachse verläuft, und das in einem die Untersuchungsachse umgebenden Bereich im Zentrum der Spulenanordnung eine Homogenität senkrecht zur Untersuchungsachse von $\Delta B/B = 10^{-4}$ besitzt. Hierbei gibt $\Delta B$ die örtliche Schwankung des Magnetfeldes B senkrecht zur Untersuchungsachse an.

Zur Erzeugung starker Magnetfelder sind aber aufgrund der begrenzten Strombelastung des zu verwendeten Wicklungsmaterials relativ große Wicklungsquerschnitte für die einzelnen Ringspulen vorzusehen. Aus Review of Scientific Instruments, Bd. 33, 1962, Seite 933-938 ist bekannt, daß insbesondere die Form und die Größe der Wicklungsquerschnitte einen Einfluß auf die Homogenität des Magnetfeldes in der Magnetspulenanordnung haben. Der in dieser Druckschrift eingeschlagene Weg zur Berechnung einer realen Vierspulenanordnung der eingangs genannten Art führt jedoch zu Zahlenwerten für die Spulenparameter, mit denen sich in den hier vorgesehenen Anwendungsfällen keine ausreichende Feldhomogenität erzielen läßt.

Aufgabe der Erfindung ist es, eine Magnetspulenanordnung zu schaffen, mit deren Hilfe starke Magnetfelder mit einer gegenüber den bekannten Anordnungen verbesserten Homogenität senkrecht zur Untersuchungsachse in einem sich möglichst weit in senkrechter Richtung zur Untersuchungsachse erstreckenden Bereich erzeugbar sind.

Diese Aufgabe wird gemäß der Erfindung dadurch gelöst, daß der mittlere Wicklungsdurchmesser der kleineren Ringspulen das 0,635-fache und die axiale Wicklungsbreite aller Ringspulen das 0,084-fache des mittleren Wicklungsdurchmessers der größeren Ringspulen beträgt, daß ferner die radiale Wicklungshöhe der größeren Ringspule das 0,162-fache, die radiale Wicklungshöhe der kleineren Ringspule nahezu das 0,100-fache, der Abstand der Mittelebene der größeren Ringspulen vom Zentralpunkt das 0,144-fache und der Abstand der Mittelebene der kleineren Ringspulen vom Zentralpunkt das 0,376-fache des mittleren Wicklungsdurchmessers der größeren Ringspulen beträgt, und daß die Amperewindungszahl der größeren Ringspulen wenigstens annähernd das 1,65-fache der Amperewindungszahl der kleineren Ringspulen ist.

Die einzelenen Ringspulen, deren Spulenebenen senkrecht zu einer durch die Mittelpunkte der Ringspulen hindurchtretenden Untersuchungsachse liegen, besitzen jeweils einen rechteckförmigen Wicklungsquerschnitt, dessen Seiten parallel bzw. senkrecht zur Untersuchungsachse verlaufen. Der mittlere Wicklungsdurchmesser einer Ringspule ergibt sich dabei als Abstand der Mittelpunkte von Wicklungsquerschnitten, die sich in einer die Untersuchungsachse enthaltenen Ebene gegenüberliegen.

Mit Hilfe einer derartigen Magnetspulenanordnung wird nun erreicht, daß das in Richtung der Untersuchungsachse und rotationssymmetrisch zu dieser verlaufende Magnetfeld bei genauer Dimensionierung und Positionierung der einzelnen Ringspulen im Zentrum der Magnetspulenanordnung bis zu einem Abstand von näherungsweise 13,9 % des größeren mittleren Wicklungsdurchmessers von der Untersuchungsachse eine Homogenität senkrecht zur Untersuchungsachse von $10^{-5}$ und bis zu einem Abstand von näherungsweise 17,3 % des größeren mittleren Wicklungsdurchmessers von der Untersuchungsachse eine Homogenität senkrecht zur Untersuchungsachse von $10^{-4}$ besitzt.

Hierbei ist wichtig, daß bei fest vorgegebenem mittleren Wicklungsdurchmesser der größeren Ringspulen der der kleineren Ringspulen bei vorgegebener Position auf der Untersuchungsachse nur um weniger als 0,3 % vom ermittelten Wert abweichen darf, während die Wickelhöhen und Wickelbreiten bis zu ± 3 % um ihre ermittelten Werte schwanken können, ohne daß hierdurch die Homogenitätseigenschaften der Magnetspulenanordnung verschlechtert werden. Hierbei ist allerdings darauf zu achten, daß das geforderte Verhältnis der Amperewindungszahlen der größeren zu den kleineren Ringsspulen wenigstens näherungsweise konstant bleibt. Die Abstände der Mittelebenen der Ringspulen vom Zentralpunkt sind hierbei durch den mittleren Wicklungsdurchmesser der größeren Ringspule festgelegt und müssen mit einer Genauigkeit von besser als 0,1 % eingehalten werden.

Der Untersuchungsbereich, also die radiale Ausdehnung des homogenen Magnetfeldbereiches im Zentrum der Magnetspulenanordnung, kann dabei durch lineare Vergrößerung oder Verkleinerung der Abmessungen der Magnetspulenanordnung (bzw. der Ringspulenabmessungen) entsprechend vergrößert oder verkleinert werden, so daß aus Gründen der Kosten- und Gewichtsersparnis eine Magnetspulenanordnung entsprechend der Größe der jeweils mit ihr zu untersuchenden Körper ausgelegt werden kann.

Die Stärke des Magnetfeldes im Untersuchungsbereich wird dabei durch die höchstzulässige Stromdichte des Leitermaterials der Ringspulenwicklungen begrenzt, die ihrerseits von der verwendeten Kühlung der Ringspulen abhängt.

Magnetspulenanordnungen dieser Art werden vorzugsweise für die Magnetresonanz-Spektroskopie verwendet, mit deren Hilfe die innere Struktur eines Körpers ermittelt werden kann. Wie bereits in der DE-1 27 55 956 beschrieben, werden die aus unterschiedlichen Bereichen des Körpers kommenden Kerninduktionssignale durch unterschiedliche Resonanzfrequenzen der Kernspins im Körper voneinander getrennt. Die Resonanzfrequenz wird dabei durch die Stärke eines den Körper durchdringenden Magnetfeldes bestimmt, das sich aus einem in Richtung der Untersuchungsachse und rotationssymmetrisch zu dieser verlaufenden, senkrecht zur Untersuchungsachse nahezu homogenen Magnetfeld und weiteren einstellbaren, magnetischen Gradientenfeldern mit konstanten Magnetfeldgradienten zusammensetzt. Vorzugsweise verlaufen hierbei zur dreidimensionalen Untersuchung des Körpers zwei senkrecht aufeinander stehende Magnetfeldgradienten jeweils senkrecht zur Richtung des homogenen Magnetfeldes, während ein dritter Magnetfeldgradient in Richtung des homogenen Magnetfeldes verläuft. Mit Hilfe des Magnetfeldes (homogenes Magnetfeld und magnetische Gradientenfelder) lassen sich nun definierte Bereiche des zu untersuchenden Körpers anregen, so daß eine eindeutige Zuordnung der erhaltenen Kerninduktionssignale zu den sie aussendenden Körperbereichen möglich ist.

Je besser nun das homogene Magnetfeld ist, umso kleiner können die diesem Feld zu überlagernden magnetischen Gradientenfelder sein, ohne daß die Eindeutigkeit zwischen Kerninduktionssignal und zugehörigem Körperbereich verloren geht. Da die Gradientenfelder periodisch dem homogenen Magnetfeld überlagert werden, ergibt sich daraus der Vorteil, daß beim Schalten der Gradientenfelder keine zu hohen Induktionsspitzen auftreten.

Durch die geringe Größe der Gradientenfelder ergibt sich ferner der weitere Vorteil, daß sich die Abklingzeit des durch Hochfrequenzimpulse periodisch angeregten Kerninduktionssignals verlängert. D.h., die exponentiell abfallende Amplitude des Kerninduktionssignals erreicht erst nach längerer Zeit eine Größe, die der Amplitude der Rauschsignale entspricht. Ein Kerninduktionssignal besitzt daher gegenüber solchen mit kurzen Abklingzeiten einen größeren Informationsinhalt, so daß ein zu untersuchender Körperbereich weniger oft angeregt zu werden braucht. Die gesamte Abtastzeit eines Körpers verkürzt sich hierdurch erheblich.

Nach einer vorteilhaften Weiterbildung der Erfindung sind die Ringspulen aus Stromleitern gewickelt, die ein rechteckförmiges Hohlprofil besitzen.

Die Ringspulen können auf diese Weise einfach gekühlt werden, indem ein Kühlmedium, z.B. Wasser, durch das Innere der Leiter geführt wird. Die Stromdichte in den Ringspulen läßt sich dann weiter vergrößern, so daß stärkere Magnetfelder im Innern der Spulenanordnung erzeugbar sind.

Die Zeichnung stellt ein Ausführungsbeispiel der Erfindung dar. Es zeigen

Fig. 1 ein Blockschaltbild eines Gerätes zur Ermittlung der inneren Körperstruktur mit Hilfe der Magnetresonanz-Spektroskopie,

Fig. 2 eine schematische Darstellung einer Magnetspulenanordnung nach der Erfindung und

Fig. 3a, b die Seiten- bzw. Vorderansicht einer mechanischen Halterung der Magnetspulenanordnung.

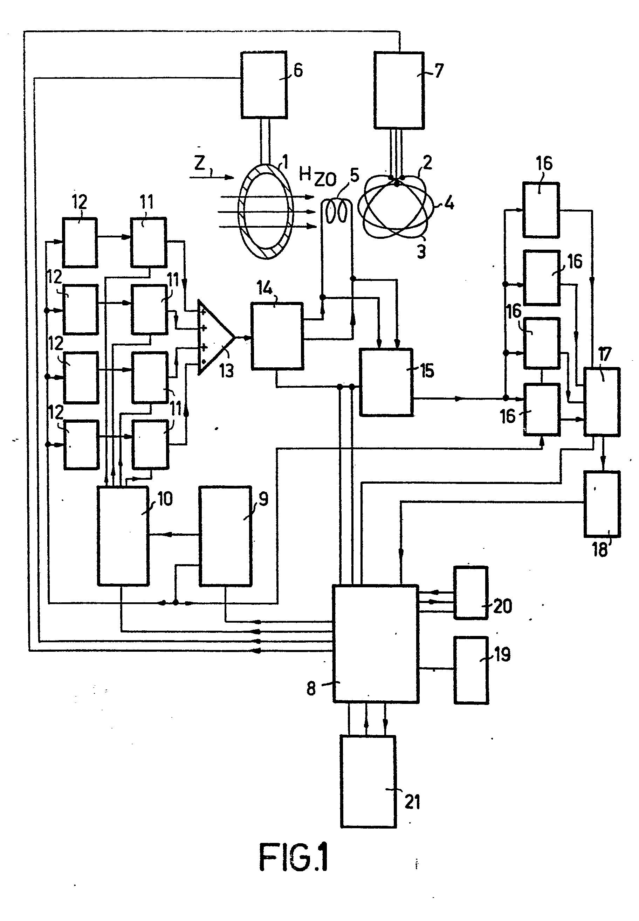

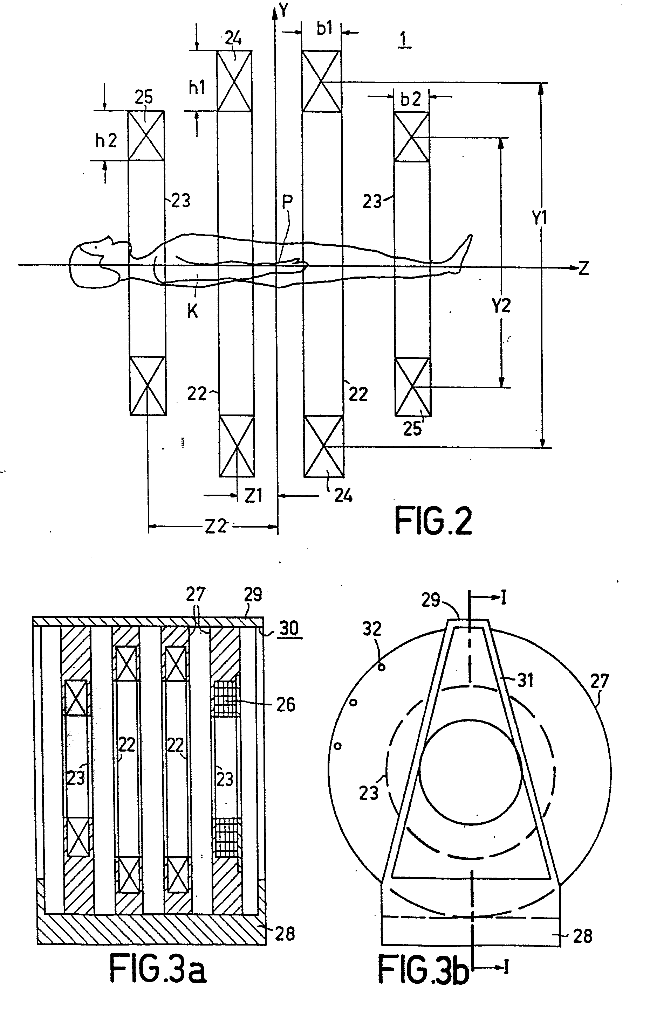

In Fig. 1 ist ein Blockschaltbild eines Gerätes zur Ermittlung der nuklearen Spindichteverteilung eines Körpers dargestellt. Hierbei erzeugt eine nur schematisch dargestellte Magnetspulenanordnung 1 (siehe Fig. 2) ein homogenes Magnetfeld Hzo, welches in Richtung einer Koordinatenachse Z eines dreidimensionalen, rechtwinkligen Koordinatensystems XYZ verläuft und einen Körper K (siehe Fig. 2), der im Innern der Magnetspulenanordnung 1 liegt, durchdringt. Der Körper K, der z.B. auf einem Patiententisch liegt, ist hierbei relativ zur Magnetspulenanordnung 1 in Richtung der Koordinatenachse Z, die die Untersuchungsachse darstellt, verschiebbar. Drei Gradientenspulen 2, 3 und 4 erzeugen hinreichend konstante magnetische Feldgradienten $\partial Hz/\partial x, \partial Hz/\partial y$ und $\partial Hz/\partial z$, wobei alle Feldgradienten getrennt einstellbar sind. Die Anregung der Kernspins und Messung der Kerninduktions (Kernresonanz-)-Signale erfolgt durch eine Hochfrequenzspule 5. Sowohl die Gradientenspulen 2, 3 und 4 als auch die Hochfrequenzspule 5 umgreifen den zu untersuchenden Körper K (Fig. 4) im Bereich der Magnetspulenanordnung. Die Stromversorgung der Magnetspulenanordnung 1 und der Gradientenspulen 2-4 erfolgt durch Netzgeräte 6 und 7, die unter der Kontrolle einer elektronischen Einheit 8 (Rechen- und Steuergerät) stehen. Die elektronische Einheit 8 steuert den gesamten Meßprozeß und dient anschließend zur Rekonstruktion von die innere Struktur des Körpers K darstellenden Bildern.

Das zur Anregung der Kernspins notwendige Hochfrequenzsignal wird von einem Frequenz-Synthetisierer 9 abgeleitet, der außerdem einen zur Erzeugung eines Modulationssignals er-

forderlichen Pulsgenerator 10 steuert. Der Pulsgenerator 10 bestimmt über das Modulationssignal mit Hilfe der Modulatoren 11 die Dauer und die Frequenzbandbreite des die Kernspins anregenden Hochfrequenzsignals, welches aus mehreren phasenverschobenen Komponenten bestehen kann, die durch die Phasenschieber 12 erzeugt werden. Im Addierer 13 werden die phasenverschobenen Komponenten des Hochfrequenzsignals addiert. Ein Hochfrequenz- (HF) Leistungsverstärker 14 führt die notwendige HF-Energie zum Anregen der Kernspins der HF-Spule 5 zu, wobei die Impulsleistung je nach Meßverfahren und Körpergröße zwischen 0,05 und 1 Kilo-Watt liegt.

Nach erfolgter Anregung der Kernspins empfängt die HF-Spule 5 das Kerninduktionssignal. Es durchläuft einen weiteren HF-Verstärker 15, anschließend erfolgt seine Demodulation in den Demodulatoren 16. Ein Analog-Digital-Wandler 17 setzt das demodulierte Kerninduktionssignal in digitale Form um. Ein nachfolgender Signalmittler 18 kann bei Wiederholung des Meßzyklus den Rauschabstand unterschiedlicher Kerninduktionssignale verbessern. Außerdem kann der Signalmittler 18 als digitaler Pufferspeicher benutzt werden.

Mit Hilfe der elektronischen Einheit 8 können dann nach bekannten Verfahren aus den Kerninduktionssignalen die gewünschten Körperbilder erzeugt werden, welche auf einem Monitor 19 darstellbar sind oder auf einen Plattenspeicher 20 in digitaler Form abgelegt werden können. Die Bedienung der gesamten Anlage erfolgt über ein Daten-Ein-Ausgabegerät 21.

In der Fig. 2 ist die Magnetspulenanordnung 1 nach der Erfindung näher dargestellt. Sie besitzt zwei innere Ringspulen 22 und zwei äußere Ringspulen 23, die mit ihren Spulenebenen senkrecht zur Untersuchungsachse Z, die durch die Spulenmittelpunkte hindurchtritt, angeordnet sind. Die Ringspulen 22 bzw. 23 liegen dabei symmetrisch zu einem auf der Untersuchungsachse festgelegten Zentralpunkt P, der beispielsweise den Ursprung des rechtwinkligen, dreidimensionalen Koordinatensystems XYZ darstellt.

Die inneren Ringspulen 22 besitzen rechteckförmige Wicklungsquerschnitte 24, deren Seiten parallel zur Untersuchungsachse Z (also in axialer Richtung) bzw. senkrecht dazu verlaufen, wobei deren axiale Wicklungsbreite b1 = 112 mm bzw. deren radiale Wicklungshöhe h1 = 216 mm beträgt. Der mittlere Wicklungsdurchmesser Y1 der größeren Ringspulen 22 beträgt 1 332 mm, während der Betrag des axialen Abstandes Z1 ihrer Mittelebenen vom Zentralpunkt P zu Z1 = 192 mm bestimmt ist. Die Mittelebene ist hierbei die Ebene, in der die Schnittpunkte der Flächendiagonalen aller Wicklungsquerschnitte einer jeweiligen Ringspule liegen.

Die äußeren Ringspulen 23 besitzen ebenfalls rechteckförmige Wicklungsquerschnitte 25, deren axiale Wicklungsbreite b2 = 112 mm und deren radiale Wicklungshöhe h2 = 128 mm beträgt. Der mittlere Wicklungsdurchmesser Y2 der Ringspulen ist hier zu Y2 = 846 mm bestimmt, während der Betrag des axialen Abstandes Z2 ihrer Mittelebenen vom Zentralpunkt P zu Z2 = 501 mm festgelegt ist.

Mit Hilfe der in Fig. 2 dargestellten Magnetspulenanordnung 1 läßt sich nun ein in Richtung der Untersuchungsachse Z und rotationssymmetrisch zu dieser verlaufendes Magnetfeld Bz erzeugen, dessen Magnetfeldstärke z.B. 0,1 Tesla betragen kann und das um den Zentralpunkt P herum bis zu einem senkrechten Abstand von näherungsweise 13,9 % (185 mm) des grösseren mittleren Wicklungsdurchmessers Y1 von der Untersuchungsachse Z eine Homogenität senkrecht zur Untersuchungsachse von $\Delta Bz/Bz = 10^{-5}$ und bis zu einem senkrechten Abstand von näherungsweise 17,3 % (230 mm) der größeren mittleren Wicklungsdurchmessers Y1 von der Untersuchungsachse eine Homogenität senkrecht zur Untersuchungsachse von $\Delta Bz/Bz = 10^{-4}$ besitzt. Hierbei ist die Amperewindungszahl der inneren größeren Ringspulen 22 etwa gleich dem 1,65-fachen der Amperewindungszahl der kleineren, äußeren Ringspulen 23.

Die erreichbare Magnetfeldstärke der Magnetspulenanordnung 1 wird durch die höchstzulässige Stromdichte des Leitermaterials der Ringspulen 22, 23 beschränkt, die ihrerseits von der Kühlung der Spulenwicklungen abhängt. Um relativ starke Magnetfelder bei nicht zu großen Wicklungsquerschnitten erzeugen zu können, werden als Leitermaterial für die Ringspulen 22, 23 Hohlprofilleiter 26 (siehe Fig. 3a) verwendet, die z.B. aus Kupfer oder Aluminium bestehen können und die beispielsweise rechteckförmigen oder quadratischen Querschnitt besitzen. Zur Kühlung der Ringspulen 22, 23 werden die Hohlprofilleiter 26 von einem Kühlmedium, z.B. von Wasser, durchströmt. Natürlich können die Ringspulen 22, 23 auch von außen, z.B. mit Hilfe von Kühlschlangen, die von einem Kühlmedium durchströmt werden und an den Ringspulen 22, 23 anliegen, gekühlt werden. Die Hohlprofilleiter 26 für die größeren bzw. kleineren Ringspulen 22, 23 besitzen beispielsweise gleiche Querschnittsabmessungen, so daß die Ringspulen 22, 23 unterschiedliche Windungszahlen aufweisen. Natürlich können für die Ringspulen 22, 23 auch Hohlprofilleiter mit unterschiedlichen Querschnittsabmessungen verwendet werden. Die erforderliche Amperewindungszahl kann dann durch Veränderung der Ströme in den zu jeweils einer Ringspule gehörenden Windungen eingestellt werden.

In Fig. 3a und b ist die Seiten- und Vorderansicht einer mechanischen Halterung der Magnetspulenanordnung 1 gezeigt. Hierbei stellt die Seitenansicht in Fig. 3a einen Schnitt gemäß der Linie I-I der Magnetspulenanordnung 1 nach Fig. 3b dar. Die in Fig. 3a dargestellten Ringspulen 22, 23 liegen konzentrisch in jeweils scheibenförmigen Spulenträgern 27, in die sie z.B.

seitlich einschiebbar sind. Die Spulenträger 27, die aus magnetisch nicht leitendem Material bestehen, dienen der Formstabilität der Ringspulen 22, 23 und zu ihrer Positionierung und sind in einer aus einem unteren und oberen Tragrahmen 28, 29 bestehenden Halterung 30, die seitliche Stützelemente 31 zur Abstützung des oberen Tragrahmens 29 besitzt, montiert. Die Spulenträger 27 haben alle den gleichen äußeren Durchmesser und sind mit dem unteren und oberen Tragrahmen 28, 29 nach erfolgter Positionierung fest verbunden. Sie besitzen ferner eine konzentrische Öffnung, deren Durchmesser dem Innendurchmesser der jeweiligen Ringspulen 22, 23 entspricht, so daß ein zu untersuchender Körper K (Fig. 2) auf einem Patiententisch liegend ins Innere der Magnetspulenanordnung 1 geschoben werden kann. Die Spulenträger 27 können ferner, wie in Fig. 3b gezeigt, untereinander durch in Richtung der Untersuchungsachse Z verlaufende Stützstangen 32 fest verbunden sein, die die Spulenträger 27 an ihrem äußeren Rand durchsetzen und auf Distanz halten.

## Ansprüche

1. Magnetspulenanordnung zur Erzeugung eines wenigstens in ihrem Zentrum homogenen Magnetfeldes, vorzugsweise für die Magnetresonanz-Spektroskopie, bestehend aus vier ebenen, paarweise gleichen Ringspulen, von denen zu verschiedenen Paaren gehörende Ringspulen, die mit ihren Spulenebenen senkrecht zu einer durch die Spulenmittelpunkte hindurchtretenden Untersuchungsachse liegen und symmetrisch zu einem Zentralpunkt auf der Untersuchungsachse angeordnet sind, unterschiedliche mittlere Wicklungsdurchmesser sowie rechteckförmige Wicklungsquerschnitte besitzen, deren Seiten senkrecht bzw. parallel zur Untersuchungsachse verlaufen, wobei das Spulenpaar mit dem größeren mittleren Wicklungsdurchmesser zwischen dem mit dem kleineren mittleren Wicklungsdurchmesser liegt und alle Ringspulen in gleicher Richtung vom Strom durchflossen werden, dadurch gekennzeichnet, daß der mittlere Wicklungsdurchmesser (Y2) der kleineren Ringspulen (23) das 0,635-fache und die axiale Wicklungsbreite (b1, b2) aller Ringspulen das 0,084-fache des mittleren Wicklungsdurchmessers (Y1) der größeren Ringspulen (22) beträgt, daß ferner die radiale Wicklungshöhe (h1) der größeren Ringspule daß 0,162-fache, die radiale Wicklungshöhe (h2) der kleineren Ringspule (23) nahezu das 0,100-fache, der Abstand (Z1) der Mittelebene der größeren Ringspulen vom Zentralpunkt (P) das 0,144-fache und der Abstand (Z2) der Mittelebene der kleineren Ringspulen vom Zentralpunkt das 0,376-fache des mittleren Wicklungsdurchmessers (Y1) der größeren Ringspulen (22) beträgt, und daß die Amperewindungszahl der größeren Ringspulen (22) wenigstens annähernd das 1,65-fache der Amperewindungszahl der kleineren Ringspulen (23) ist.

2. Magnetspulenanordnung nach Anspruch 1, dadurch gekennzeichnet, daß die Ringspulen (22, 23) aus Stromleitern (26) gewickelt sind, die ein rechteckförmiges Hohlprofil besitzen.

## Claims

1. A magnet coil arrangement for producing a magnetic field which is homogeneous at least at ist centre, preferably for magnetic resonance spectroscopy, consisting of four flat, pair-wise identical ring coils, those ring coils belonging to different pairs whose coil planes extend perpendicularly to an axis of examination passing through the coil centres and are arranged symmetrically with respect to a central point on the axis of examination, having different mean winding diameters as well as rectangular winding cross-sections whose sides extend perpendicularly and parallel, respectively, to the axis of examination, the pair of coils having the larger mean winding diameter being situated between those having the smaller mean winding diameter, and current being made to flow in the same direction through all the ring coils, characterized in that the mean winding diameter (Y2) of the smaller ring coils (23) amounts to 0,635 times the mean winding diameter (Y1) of the larger ring coils (22), the axial winding width (b1, b2) of all ring coils amounting to 0,084 times this diameter, the radial winding height (h1) of the larger ring coils amounting to 0.162 times the mean winding diameter (Y1) of the larger ring coils (22), the radial winding height (h2) of the smaller ring coils (23) amounting to 0.100 times this diameter, the distance (Z1) between the central plane of the larger ring coils and the central point (P) amounting to 0.144 times this diameter, and the distance (Z2) between the central plane of the smaller ring coils and the central point amounting to 0.376 times this diameter, the number of ampereturns of the larger ring coils (22) being at least approximately 1.65 times the number of ampereturns of the smaller ring coils (23).

2. A magnet coil arrangement as claimed in Claim 1 characterized in that the ring coils (22, 23) are wound from current conductors (26) having a rectangular, hollow section.

## Revendications

1. Structure de bobine magnétique servant à engendrer un champ magnétique dont au moins le centre est homogène, de préférence pour la spectroscopie de résonance magnétique, constituée par quatre bobines annulaires planes identiques par paires, parmi lesquelles des bobines annulaires appartenant à des paires différentes et dont les plans de bobine s'étendent perpendiculairement à un axe d'examen passant par le centre des bobines et sont disposés symétriquement par rapport à un point central sur l'axe d'examen, présentent des diamètres d'enroulement moyens différents aussi bien que des sections transversales rectangulaires, dont les côtés s'étendent perpendiculairement, respectivement, parallèlement à l'axe d'examen, la paire de bobines présentant le plus grand diamètre d'enroulement moyen se trouvant entre celle présentant le plus petit diamètre d'enroulement moyen et toutes les bobines annulaires étant traversées dans la même direction par du courant, caractérisée en ce que le diamètre d'enroulement moyen $Y_2$ des plus petites bobines annulaires (23) est égal à 0,635 fois et la largeur d'enroulement axiale ($b_1$, $b_2$) de toutes les bobines annulaires égale à 0,084 fois le diamètre d'enroulement moyen ($Y_1$) des plus grandes bobines annulaires (22), puis que la hauteur d'enroulement radiale ($h_1$) des plus grandes bobines annulaires est égale à 0,162 fois, la hauteur d'enroulement radiale ($h_2$) des plus petites bobines annulaires (23) pratiquement égale à 0,100 fois, la distance ($Z_1$) comprise entre le plan médian des plus grandes bobines annulaires et le point central (P) égal à 0,144 fois et la distance ($Z_2$) comprise entre le plan médian des plus petites bobines annulaires et le point central égal à 0,376 fois le diamètre d'enroulement moyen ($Y_1$) des plus grandes bobines annulaires (22), et que le nombre d'ampère-tours des plus grandes bobines annulaires (22) est égal, au moins approximativement, à 0,165 fois le nombre d'ampère-tours des plus petites bobines annulaires (23).

2. Structure de bobine magnétique selon la revendication 1, caractérisée en ce que les bobines annulaires (22, 23) sont constituées par enroulement de conducteur de courant (26) présentant un profil creux rectangulaire.

FIG.1

FIG.2

FIG.3a

FIG.3b